# FASCICULE DE BREVET EUROPEEN

(11) **EP 2 337 796 B1**
(45) Date de publication et mention de la délivrance du brevet: **17.04.2013**
(21) Numéro de dépôt: 09736873.2
(22) Date de dépôt: 22.09.2009
(51) Int. Cl.: C07K 14/78, C07K 16/18, A61K 38/04

(54) **PROTEINES RECOMBINANTES A ACTIVITE HEMOSTATIQUE CAPABLES D'INDUIRE L'AGREGATION PLAQUETT AIRE**
REKOMBINANTE PROTEINE MIT HÄMOSTATISCHER WIRKUNG UND DER FÄHIGKEIT, DIE THROMBOZYTENAGGREGATION ZU INDUZIEREN
RECOMBINANT PROTEINS HAVING HAEMOSTATIC ACTIVITY AND CAPABLE OF INDUCING PLATELET AGGREGATION

(30) Priorité: 24.09.2008 FR 0856423
(43) Date de publication de la demande: 29.06.2011
(73) Titulaire: Centre Hospitalier Universitaire De Dijon, 21000 Dijon (FR)
(72) Inventeur: VANDROUX, David, 21000 Dijon (FR); DE MAISTRE, Emmanuel, 21121 Fontaine-les-Dijon (FR); PROST, Edourd, 21000 Dijon (FR)
(74) Mandataire: Regimbeau
(86) Numéro de dépôt international: PCT/EP2009/062271
(87) Numéro de publication internationale: WO 2010/034718

(56) Documents cités:
- WO-A-01/34647
- US-A1- 2004 151 731
- P DAVID TOMAN & B DE CROMBRUGGHE: "The mouse type-III procollagen-encoding gene: genomic cloning and complete DNA sequence" GENE., vol. 147, 1994, pages 161-168, XP023540937 ELSEVIER, AMSTERDAM.

### SEQUENCE LISTING

<110> Centre Hospitalier Universitaire Dijon VANDROUX, David DE MAISTRE, Emmanuel PROST, Edouard
<120> Protéines recombinantes à activité hémostatique capables d'induire l'agrégation plaquettaire
<130> D26270
<150> FR 0856423
   <151> 2008-09-24
<160> 8
<170> PatentIn version 3.3
<210> 1
   <211> 152
   <212> PRT
   <213> Artificial
<220>
   <223> protéine recombinante collIII-like
<400> 1
   Met Met Ser Phe Val Gln Lys Gly Ser Trp Leu Leu Leu Ala Leu Leu
   1               5                   10                  15
   His Pro Thr Ile Ile Leu Ala Gln Gly Arg Pro Gly Ala Pro Gly Glu
               20                  25                  30
   Arg Gly Leu Pro Gly Pro Pro Gly Pro Arg Gly Ala Ala Gly Glu Pro
           35                  40                  45
   Gly Arg Asp Gly Val Pro Gly Gly Pro Gly Met Arg Gly Met Pro Gly
       50                  55                  60
   Ser Pro Gly Gly Pro Gly Ser Asp Gly Lys Pro Gly Pro Pro Gly Ser
   65                  70                  75                  80
   Gln Gly Glu Ser Gly Arg Pro Gly Pro Pro Gly Glu Asn Gly Lys Pro
                   85                  90                  95
   Gly Glu Pro Gly Pro Lys Gly Asp Ala Gly Pro Pro Gly Pro Pro Gly
               100                 105                 110
   Pro Pro Gly Pro Pro Gly Pro Pro Gly Pro Pro Gly Pro Pro Gly Pro
           115                 120                 125
   Pro Gly Pro Pro Gly Pro Pro Gly Pro Arg Gly Asp Lys Gly Pro Pro
       130                 135                 140
   Gly Ala Pro Gly Pro Cys Pro Gly
   145                 150
<210> 2
   <211> 459
   <212> DNA
   <213> Artificial
<220>
   <223> Séquence codant pour protéine recombinante collIII-like
<400> 2
   atgatgagct tcgtgcagaa ggggagctgg ctgcttctcg ctctgcttca tcctactatt 60
   atcctggcac aggggcgccc cggagctcct ggagagagag gattgcctgg acctccaggg 120
   cccagaggag ctgctggaga acctggcaga gatggcgtcc ctggaggacc aggaatgagg 180
   ggcatgcccg gaagcccagg aggaccagga agcgatggga agccagggcc tcccggaagc 240
   cagggagaaa gcggcagacc aggacctcct ggagagaacg gaaagcctgg agaaccaggc 300
   ccaaagggag atgccggacc acctggccca ccaggccctc ccggaccacc tggacctcca 360
   ggccctcctg gccctcctgg acctcctgga ccacctggac ctccaggccc aagaggcgac 420
   aagggacctc ctggagcccc tggaccttgc cctggctaa 459
<210> 3
   <211> 21
   <212> DNA
   <213> Artificial
<220>
   <223> amorce S
<400> 3
   agctggcgcg ccgccaccat g 21
<210> 4
   <211> 27
   <212> DNA
   <213> Artificial
<220>
   <223> amorce AS
<400> 4
   gcttccggga ggccctggct tcccatc 27
<210> 5
   <211> 69
   <212> DNA
   <213> Artificial
<220>
   <223> amorce tagNVH001-sens
<400> 5
   gctgccatgg gcagcagcca tcatcatcat catcacggtc gcccgggagc tcctggagag 60
   agaggattg 69
<210> 6
   <211> 38
   <212> DNA
   <213> Artificial
<220>
   <223> amorce tagNVH001-antisens
<400> 6
   gcacggatcc tattagccag ggcaaggtcc aggggctc 38
<210> 7
   <211> 6
   <212> PRT
   <213> Artificial
<220>
   <223> Peptide motif
<400> 7
   Gly Ala Pro Gly Glu Arg
   1 5
<210> 8
   <211> 8
   <212> PRT
   <213> Artificial
<220>
   <223> peptide motif
<400> 8
   Lys Pro Gly Glu Pro Gly Pro Lys
   1 5

## Description

La présente invention concerne des protéines recombinantes constituées de l'assemblage de séquences peptidiques décrites pour interagir avec les récepteurs plaquettaires aux collagènes. Ces protéines ont une activité pro-agrégante, indépendante de la formation d'une triple hélice. Elles peuvent être produites en bactéries et en cellules de mammifères.

Les collagènes sont les constituants structuraux principaux de la matrice extracellulaire de tous les organismes multicellulaires. Il s'agit d'une famille de protéines composée de 28 types différents jouant un rôle au cours du développement et dans l'homéostasie tissulaire. Ils sont capables de s'assembler en différentes structures supramoléculaires sous forme de fibrilles, de microfibrilles ou encore de réseaux.

Les collagènes présentent la caractéristique commune de contenir un ou plusieurs domaine(s) ayant une structure en triple hélice formé de trois chaînes polypeptidiques, ou chaînes α, enroulées les unes aux autres. Cette caractéristique est permise par la présence, tous les trois acides aminés, d'une glycine au niveau des motifs hélicoïdaux qui sont constitués de séquences répétées de type G-X-Y où X est souvent une proline et Y une hydroxyproline. Cette hydroxyproline est essentielle à la stabilisation de la triple hélice et est caractéristique des collagènes. Les résidus prolines sont hydroxylés essentiellement par la prolyl-4-hydroxylase (P-4-H) en 4-hydroxyproline. Il existe une deuxième hydroxylase, la prolyl-3-hydroxylase qui permet l'hydroxylation de la proline lorsque celle-ci est en position X, alors qu'en position Y se trouve déjà une proline hydroxylée par la P-4-H. Dans une molécule de collagène, les chaînes alpha peuvent être identiques ou toutes différentes.

Les molécules de collagène sont formées de domaines hélicoïdaux (ou domaines collagènes) encadrés de domaines non hélicoïdaux, nommés N et C propeptides. La reconnaissance des trois chaînes α formant une molécule et le début de leur assemblage sont sous le contrôle de l'extrémité C-terminale (C-propeptide). Elle est réalisée au niveau du réticulum endoplasmique. Par la suite, les N et C propeptides sont excisés au cours de la maturation du collagène, laissant subsister de courtes séquences non hélicoïdales, les télopeptides.

Les collagènes jouent un rôle important au niveau des parois des vaisseaux en maintenant leur intégrité et leur élasticité. Cette paroi est formée des collagènes de types I et III, fibrillaires, et du collagène de type IV, en réseau. Il est à noter que le collagène de type III est également fortement exprimé au niveau des plaques d'athérome. Par ailleurs, les collagènes sont capables de moduler les fonctions de certaines cellules par interaction directe avec des récepteurs cellulaires spécifiques. C'est ainsi que les collagènes, principalement les types I et III, sont de puissants activateurs de la fonction plaquettaire.

Le collagène de type III est un homotrimère constitué de 3 chaînes α1 agencées en triple hélice. Deux triplets G-P-P (potentiellement hydroxylées), présents à l'extrémité C-terminale des chaînes α1, seraient ensuite suffisants et nécessaires à la nucléation et au repliement de la triple hélice (Bulleid et αl., EMBO J. 1997 Nov 17; 16(22) : 6694-701). En revanche, ces deux triplets ne sont pas suffisants pour permettre l'association initiale des trois chaînes. Il est intéressant de noter que Bulleid et coll. (1997) ont observé une meilleure efficacité de formation de la triple hélice lorsque trois triplets sont maintenus, cette efficacité étant même plus élevée que pour la molécule sauvage. Plusieurs protéines sont impliquées dans le processus d'assemblage des chaînes monomériques : la HSP47 (Heat Shock Protein) et la PDI (Protein Disulfide Isomerase). Le propeptide-C, situé à l'extrémité C terminale des chaînes monomériques, apparaît impliqué dans l'alignement des chaînes α et dans la formation des ponts disulfures nécessaires à la stabilisation de la protéine (Bulleid et al., EMBO J. 1997 Nov 17; 16(22) : 6694-701). Ce propeptide-C serait donc requis uniquement pour assurer l'association des chaînes monomériques. Différents points sont à retenir le concernant :
- Il est constitué d'une séquence discontinue de 15 aa qui détermine l'assemblage type spécifique des chaînes α1 (Hulmes DJ, J Struct Biol. 2002 Jan-Feb; 137(1-2) : 2-10*.*).
- Un domaine « coiled-coil » situé au début du propeptide-C est impliqué dans la trimérisation du collagène III (Bulleid et al., EMBO J. 1997 Nov 17; 16(22) : 6694-701). Ce domaine est constitué de 4 heptapeptides (McAlinden et coll., J Biol Chem. 2003 Oct 24; 278(43) : 42200-7).
- Il contient 8 résidus cystéine qui permettent la formation de ponts disulfures intra-et inter-caténaires.

La présence de ponts disulfures entre les chaînes au niveau du télopeptide-C ou du propeptide-C n'est pas requise pour l'association des chaînes et la formation de la triple hélice (Bulleid et coll., Biochem J. 1996 Jul 1; 317 (Pt 1) : 195-202.). Il est à noter que les expériences sont menées avec le propeptide-N. Les notions de trimère et de triple hélice alpha apparaissent paradoxalement comme indépendantes. En l'absence de propeptide-N, il semble judicieux de laisser soit la cystéine 2 du propeptide-C, soit les deux cystéines du télopeptide-C. Ce dernier motif, nommé « the knot sequence » (GPCCG), permettrait la formation des ponts disulfures uniquement grâce à un phénomène préalable d'assemblage et de repliement des chaînes αl (Boudko et Engel, J Mol Biol. 2004 Jan 30; 335(5) : 1289-97).

Qu'elle soit d'origine traumatique ou la conséquence de l'athérosclérose, l'atteinte de la paroi artérielle s'accompagne de la destruction de l'endothélium vasculaire et l'exposition de composants thrombogènes tels que le collagène. Il s'en suit l'adhésion de plaquettes au niveau du site lésé, au contact de surfaces riches en collagène ou en fragments de collagène, leur activation et la formation d'un thrombus. L'adhésion et la stabilisation des plaquettes au contact de ce collagène sont permises par des interactions multiples, de hautes affinités, entre des récepteurs présents à la surface des plaquettes et le collagène. Cette adhésion peut intervenir indirectement suite à la liaison du domaine A1 du facteur von Willebrand (vWF) avec le complexe plaquettaire formé des glycoprotéines (Gp) GpIb-V-IX, lui-même lié au collagène par son domaine A3, ou par interaction directe entre un récepteur plaquettaire et le collagène. Plusieurs récepteurs peuvent lier des molécules de collagène au niveau de séquences peptidiques très spécifiques. Il s'agit de l'intégrine α2β1, qui joue un rôle important dans la stabilisation de la plaquette au contact du collagène, et de GpVI, considéré comme le récepteur le plus important pour l'activation plaquettaire. Un autre récepteur, TIIICBP (type III collagen-binding protein), a été décrit comme capable de lier directement le collagène.

La vitesse du flux au niveau des vaisseaux est un déterminant majeur définissant le type de récepteurs plaquettaires au collagène recruté. A vitesse de flux élevée, les plaquettes interagissent avec le collagène par l'intermédiaire du vWF *via* le récepteur GpIb puis elles sont activées par la fixation *via* GpVI, l'intégrine α2β1 n'intervenant que comme stabilisateur de la liaison plaquette-collagène. A vitesse de flux lente, les plaquettes se lient au collagène par l'intermédiaire de l'intégrine α2β1 qui est suivie de la fixation à GpVI conduisant à leur activation qui est l'étape qui prépare à l'agrégation plaquettaire. De même, d'autres récepteurs plaquettaires au collagène seraient impliqués comme TIIICBP. Dans tous les cas, GpVI joue un rôle majeur dans l'activation des plaquettes.

L'adhésion plaquettaire est un processus qui est maintenant dissocié de l'activation plaquettaire. En effet, de nombreux peptides, correspondants à de courts motifs peptidiques de collagène, sont capables d'induire l'adhésion des plaquettes sans conduire à leur activation. Certains de ces peptides présentent néanmoins la capacité d'induire l'activation des plaquettes et présentent une activité dite pro-agrégante. Leur structuration sous forme de triple hélice semble être un pré-requis indispensable pour cette activité. A noter que lorsque certains de ces peptides restent sous forme monomérique, ils présentent une activité anti-agrégante par un mécanisme qui reste à identifier mais qui pourrait être l'occupation des sites qui deviennent non disponibles pour le collagène natif.

La plupart des travaux visant à identifier les séquences peptidiques impliquées dans l'adhésion des plaquettes au collagène de type III utilise le fragment α1(III)CB4 correspondant au fragment de digestion par le CNBr présentant la plus forte activité d'agrégation.

Différents motifs peptidiques ont été décrits ces dernières années comme étant capables de lier et d'activer les plaquettes. Ils ont été testés sous forme de peptides pouvant être de deux natures :
- apparentés au collagène, formés de la répétition de motifs conservés GPO répétés n fois, non présents dans la séquence native du collagène de type III.
- ou correspondrent à des peptides formés des motifs peptidiques présents au niveau de la séquence α1(III)CB4, principalement obtenus par synthèse chimique *(*Farndale et coll., Biochem Soc Trans. 2008 Apr; 36(Pt 2) : 241-50).

L'intégrine α2β1 est un récepteur pour les collagènes, la laminine et d'autres ligands dans différents types cellulaires dont les cellules endothéliales et les plaquettes. α2β1 lie le collagène par l'intermédiaire de son domaine 1 au niveau de motifs peptidiques présents dans la séquence des collagènes fibrillaires. Différents motifs ont été décrits avec la plus forte affinité pour GFOGER présent au niveau de la chaîne α1 du collagène de type I. Pour le collagène de type III, et contrairement au collagène de type I, il semble que plusieurs motifs de type GXYGER soient nécessaires pour une liaison optimale, avec GLOGER, GMOGER, GROGER et GAOGER comme principaux motifs (Kim et coll., J Biol Chem. 2005 Sep 16; 280(37): 32512-20). D'autres motifs tels que GLOGEN et GLKGEN ont été décrits mais présentent une affinité faible pour α2β1 (Raynal et coll., J Biol Chem. 2006 Feb 17; 281(7) : 3821-31). Il est à noter que l'ensemble des peptides synthétisés à partir de ces motifs présente une activité d'adhésion que lorsqu'ils sont organisés sous forme de triple hélice.

La glycoprotéine VI (GPVI) est une glycoprotéine transmembranaire de type I de 60-65 kDa appartenant à la superfamille des immunoglobulines (Ig). Elle est constitutivement exprimée à la surface des plaquettes sous forme de complexe non covalent avec la chaîne γ commune aux récepteurs des Ig (FcRy). Les peptides décrits pour interagir avec ce récepteur sont des peptides apparentés au collagène formés de la répétition de 4 à 10 fois du triplet GPO (Morton et coll., Biochem J. 1995 Mar 1; 306 (Pt 2) : 337-44 *et* Smethurst et coll., J Biol Chem. 2007 Jan 12; 282(2) : 1296-304). Bien que formé de 10% de motif GPO, le nombre maximal de répétitions de ce triplet ne dépasse pas trois dans la séquence native du collagène de type III. Il faut noter ici encore que la formation de la triple hélice ou d'une forme polymérique, obtenue chimiquement suite à une modification des résidus cystéine ou lysine, est indispensable pour conférer à ces peptides une activité pro-agégante. De plus, la présence d'une hydroxyproline en position 3 est indispensable à cette activité. A l'inverse, lorsqu'ils sont sous forme monomérique, ces peptides présentent une activité anti-agrégante (Asselin et coll., Biochem J. 1999 Apr 15; 339 (Pt 2) : 413-8). Récemment, Jarvis et coll. ont identifié le motif peptidique dans la séquence du collagène de type III présentant l'activité d'adhésion la plus forte. Il est composé des résidus GAOGLRGGAGPOGPEGGKGAAGPOGPO localisés au niveau des acides aminés 523 à 549 de α(III) CB4 (Jarvis et coll., Blood. 2008 May 15; 111 (10) : 4986-96). Ce motif peptidique est composé de 3 GPO, qui sont non consécutifs et qui semble être le nombre minimal nécessaire pour une bonne interaction avec GpVI.

L'interaction indirecte entre le récepteur plaquettaire GpIb et le collagène est dépendante du facteur vWF. Ce vWF est une protéine multimérique plasmatique sécrétée par les cellules endothéliales et les plaquettes en réponse à des dommages vasculaires ou suite à une augmentation des contraintes parétiales. Il joue un rôle majeur dans le recrutement des plaquettes au niveau des sites lésés des territoires vasculaires soumis à des vitesses de flux élevées. Ce facteur est composé de trois domaines nommés A1, A2 et A3. Il se fixe au collagène par l'intermédiaire de son domaine A3 et lie le récepteur GpIb par son domaine A1. Le collagène de type III semble posséder un site unique, de haute affinité pour le domaine A3 du vWF, qui est également présent dans le collagène de type II. Ce motif peptidique est localisé entre les acides aminés 403 à 413 et se compose de GPRGQOVMGFO avec certains acides aminés critiques pour la fixation au vWF (Lisman T et coll., Blood. 2006 Dec 1; 108(12) : 3753-6). Verkleij et coll. ont eux identifié comme potentiel site de liaison, les acides aminés 541 à 558 composés de GAAGPOGPOGSAGTOGLQ (Verkleij et coll., Blood. 1998 May 15; 91 (10) : 3808-16). Ce motif peptidique est localisé entre le motif vWF décrit par Lisman et coll. et le motif de liaison à l'intégrine α1 β2 GMOGER.

L'équipe de Fauvel-Lafève a décrit l'existence d'un octapeptide, KOGEOGPK, localisé entre les acides aminés 655 à 662 du fragment α1(III) CB4. Le récepteur plaquettaire reconnu par cet octapeptide a été identifié et nommé TIIICBP (type III collagen binding protein) (Monnet E et coll., J Biol Chem. 2000 Apr 14; 275(15) : 10912-7). Cet octapeptide est capable d'inhiber l'interaction des plaquettes avec le collagène de type III, mais pas avec le collagène de type I, à la fois en condition statique et en condition de flux. Plus récemment, Pires et coll. ont montré que cet octapeptide possède une activité inhibitrice de l'agrégation uniquement lorsqu'il est sous forme d'homotrimère. Au contraire, sa structuration sous forme d'une triple-hélice par l'ajout à ces extrémités de cystéines et de GPP lui confère une activité pro-agrégante (Pires et coll., Eur J Med Chem. 2007 May; 42(5) : 694-701).

Les propriétés biologiques et ultrastructurales des collagènes, et notamment leur capacité de liaison à des récepteurs membranaires, ouvrent un champ d'applications important du fait de leur rôle multiple au niveau des tissus. Cependant, ces applications n'ont de sens que si il est possible de disposer de préparations homogènes de ces collagènes en quantités importantes et ce de façon reproductible.

Deux modes de production ont été utilisés à cette fin. Les travaux et les développements conduits dans ces deux domaines le sont généralement de façon très ciblée pour une application donnée. C'est ainsi que la synthèse chimique permet de produire de courts peptides ne représentant qu'une partie infime de la protéine, en général des motifs peptidiques d'intérêt. Son application principale concerne l'hémostase et plus précisément la modulation de la liaison du collagène aux plaquettes.

Cependant, ces peptides possèdent en général une seule des activités recherchées et celle-ci peut dépendre de la conformation tridimensionnelle du peptide et notamment de la formation d'une triple hélice. Ainsi, il existe un besoin important de développement de protéines recombinantes de collagènes fonctionnalisées pouvant être produites par des systèmes biologiques offrant une haute productivité. Un obstacle majeur étant les difficultés de production et de purification de ces protéines dérivées du collagène en raison de leur tendance à l'agrégation et à l'adhésion.

A ce jour, les courtes séquences peptidiques (inférieures à 50 acides aminés) ont toutes été produites par synthèse chimique et non *via* des systèmes de production cellulaire (Farndale et coll., Biochem Soc Trans. 2008 Apr; 36(Pt 2) : 241-50). De plus, ces motifs ont été synthétisés de façon isolée. A l'inverse, la synthèse de collagène de type III recombinant, présentant une activité de liaison aux plaquettes, a été réalisée *via* des systèmes de production cellulaire et c'est la totalité de la séquence, par exemple le proαl(III), qui a été utilisée. L'objectif recherché est la synthèse d'un procollagène entier capable de s'organiser en triple hélice (WO9307889). Ces collagènes recombinants ont par principe de multiples applications, celles portées par la séquence totale du collagène. WO-A-0134647(Fibrogen) décrit une portion du collagène III beaucoup plus longue que Séq. no 1.

Il s'agit donc de synthétiser des protéines de structure plus simple, plus petites et donc moins exigeantes en terme de production mais conservant les activités biologiques d'intérêt de la protéine native.

L'invention concerne ainsi des protéines recombinantes possédant les motifs nécessaires pour obtenir une activité pro-agrégante, synthétisée sous forme d'un monomère incapable de se structurer sous forme de triple hélice. L'impossibilité de formation de cette triple hélice est la conséquence de l'absence : i) des motifs de reconnaissance des chaînes α entre elles, ii) des domaines N- et C-terminaux non hélicoïdaux des collagènes ainsi que iii) des deux triplets GPO nécessaires à l'amorçage de la triple hélice. De façon surprenante, l'activité pro-agrégante est obtenue en l'absence de formation de triple hélice.

La demanderesse a montré de façon surprenante qu'il est possible de regrouper trois types de séquences peptidiques au sein d'une seule et même protéine, de type monomérique, ayant une activité pro-agrégante.

Les protéines recombinantes de la présente invention regroupent ainsi :
- des motifs peptidiques présentant au minimum une répétition de 4 triplets GPO,
- des séquences peptidiques décrites comme ayant une activité de liaison aux différents récepteurs plaquettaires présentes au niveau de la séquence native des collagènes,
- des séquences de liaison entre ces motifs formés de la répétition de triplets GXY.

La production de ces protéines par des bactéries et des cellules de mammifères permet de disposer en quantités importantes de ces collagènes recombinants de nouvelle génération, fruits de l'association de séquences peptidiques apparentées au collagène et de séquences présentent dans la forme native des collagènes. Ce type de protéines ne nécessite pas de privilégier des cellules qui ne produisent pas de façon constitutive des collagènes comme cellules hôtes pour cette production. En effet, leur séquence ne permet pas la reconnaissance des chaînes alpha des différents collagènes natifs. Au contraire, il est nécessaire de privilégier des cellules productrices de collagènes qui expriment des enzymes clés de la maturation des collagènes telles que la prolyl-4 hydroxylase (P4H) et la HSP47. Les systèmes de production de collagènes recombinants actuels utilisent au contraire des cellules non productrices naturelles de collagène (Olsen et coll., Adv Drug Deliv Rev. 2003 Nov 28; 55(12) : 1547-67 *et* Ruggiero et Koch, Methods. 2008 May; 45(1) : 75-85). Cela implique de co-transfecter le gène codant pour les enzymes comme la P4H.

Les protéines recombinantes selon l'invention présentent de nombreux avantages, dont :
- le regroupement des motifs d'intérêt au sein de protéines de petite taille , plus faciles à produire à l'aide de systèmes cellulaires de production,
- une activité pro agrégante en l'absence d'une structure en triple hélice,
- l'absence dans leur séquence des sites de reconnaissance des collagénases mais la présence de ceux pour la HSP47, permettant leur production par des cellules productrices de collagènes,
- leur activité biologique démontrée par des tests de référence (tests d'agrégation plaquettaire) qui sont utilisés pour la surveillance des traitements anti-plaquettaires actuels et l'exploration des fonctions plaquettaires.

Les propriétés biologiques des protéines recombinantes selon l'invention permettent d'envisager de nombreuses applications.

La première application concerne l'utilisation de ces protéines comme outil diagnostic dans l'exploration de la maladie thrombotique : tests d'agrégation plaquettaire, modèles de thrombose *in vitro* et évaluation de l'efficacité des traitements antiplaquettaires actuels (aspirine, clopidogrel) et à venir. Les protéines produites sont dérivées de protéines humaines et présentent dans leur séquence des sites de liaison pour certains récepteurs plaquettaires décrits pour leur rôle dans l'adhésion et l'activation plaquettaire. Les tests actuels utilisent des collagènes d'origine animale dont les séquences ne présentent pas toutes une homologie parfaite avec l'homme. Par ailleurs, la variation du nombre et de la localisation des sites d'interaction avec les récepteurs plaquettaires entre les différentes protéines permet de proposer des tests de validation de cible plus pertinents. Il existe des fragments peptidiques permettant de travailler plus spécifiquement sur chacun des récepteurs plaquettaires mais leur mode de production est différent du notre, avec une synthèse chimique vs une production naturelle par des cellules (importance des modifications post-traductionnelles).

La présente invention peut intervenir dans la composition de pansements hémostatiques (renforçant ainsi le recrutement sur site des plaquettes en cas de lésion vasculaire), ou de colles hémostatiques pour prévenir le risque de saignement (contexte chirurgical). Des produits sont actuellement commercialisés, mais avec un collagène de source animale.

Une autre application est l'utilisation de ces protéines pour activer la cicatrisation. La cicatrisation de certaines plaies nécessite la migration, l'adhésion et la différenciation sur site de certaines populations cellulaires, dont les plaquettes. Ces processus sont dépendants des intégrines de surface de ces cellules. Certains des motifs peptidiques présents dans la structure de nos protéines sont capables d'interagir avec ces intégrines en favorisant ainsi la cicatrisation.

Dans un domaine très proche, ces protéines recombinantes pourraient être proposées dans la composition de certaines crèmes dermatologiques.

Une autre application des protéines inhibitrices de la fonction plaquettaire est le traitement de l'athérothrombose. Les récepteurs plaquettaires sont des cibles thérapeutiques d'avenir pour lutter contre les complications de la maladie athérothrombotique. A ce jour, il n'existe aucune molécule capable de bloquer la phase initiale d'adhésion des plaquettes au sous-endothélium. Les différentes structures protéiques de nos protéines permettent leur interaction avec ces récepteurs conduisant à des effets antiadhésion plaquettaire (masquage des sites) et/ou pro-adhésion plaquettaire. Les différents effets peuvent être obtenus avec la même protéine en modulant ses propriétés physicochimiques.

Cette invention permet donc la mise à disposition d'un réactif biologique présentant de nombreuses applications pouvant rentrer dans la composition de trousses : pour l'évaluation des fonctions plaquettaires, pour activer la différenciation cellulaire pour le diagnostic de dysfonctions hématologiques, pour détecter par imagerie des zones de fibrose. Cette invention peut également entrer dans la composition : de pansements hémostatiques pour le recrutement sur site des plaquettes (dans le cas de lésion vasculaire), de colles hémostatiques pour prévenir le risque de saignement, ou encore dans des crèmes dermatologiques.

### Description des Séquences

SEQ ID No. 1 Protéine recombinante colIII-like
SEQ ID No.2 : Polynucléotide codant pour la protéine recombinante collIII-like
SEQ ID Nos. 3-6 : Amorces

### Description de l'invention

L'invention concerne des polypeptides isolés ayant la séquence du polypeptide de la SEQ ID No.1 ou du polypeptide de la position 25 à la position 152 de la SEQ ID No. 1.

L'invention a également pour objet des polypeptides isolés présentant au moins 70% d'identité sur toute sa longueur avec le polypeptide de la SEQ ID No.1 ou avec le polypeptide de la position 25 à la position 152 de la SEQ ID No. 1, et capables d'induire une agrégation des plaquettes sanguines humaines supérieure à 30% dans un thromboagrégomètre à 37°C avec une agitation de 1000 rpm.

Dans un mode de réalisation préféré, ces polypeptides comprennent les motifs peptidiques suivants :
- GX₁X₂GER dans lequel X₁ et X₂ représentent indépendamment un acide aminé choisi parmi A, R, N, D, Q, E, G, H, 1, K, M, F, P, S, T, W, Y, V et O ;
- (GPX₃)ₙ avec n compris entre 4 et 10 et X₃ représente P ou O ;
- GPRGQX₄GVMGFX₅ où X₄ et X₅ représentent indépendamment P ou O.
P est la proline et O est l'hydroxyproline.

Dans un autre mode de réalisation préféré, ces polypeptides comprennent les motifs peptidiques suivants :
- GAPGER,
- KPGEPGPK,
- (GPP)ₙ avec n compris entre 4 et 10,
- RGD.

Un autre objet de l'invention est un polynucléotide isolé caractérisé en ce qu'il code pour un polypeptide selon l'invention.

Dans un mode de réalisation préféré, le polynucléotide a la séquence du polynucléotide de la SEQ ID No. 2.

Un autre objet de l'invention est un anticorps se liant spécifiquement au polypeptide de la SEQ ID No. 1.

L'invention se rapporte aussi à des cassettes d'expression comprenant dans le sens de la transcription:
- un promoteur fonctionnel dans un organisme hôte,
- un polynucléotide selon l'invention,
- une séquence terminatrice fonctionnelle dans le même organisme hôte.

Un autre objet de l'invention est un vecteur comprenant un polynucléotide selon l'invention et/ou une cassette d'expression selon l'invention.

Un autre objet de l'invention est un organisme hôte transformé avec un polynucléotide selon l'invention, une cassette d'expression selon l'invention et/ou un vecteur selon l'invention.

L'invention se rapporte aussi à des compositions pour utilisation comme médicament comprenant un polypeptide selon l'invention, un polynucléotide selon l'invention, une cassette d'expression selon l'invention, un vecteur selon l'invention et/ou un organisme hôte selon l'invention.

Dans un mode de réalisation préféré, l'invention concerne des compositions pour le traitement des maladies thrombotiques.

Dans un autre mode de réalisation préféré, l'invention concerne des compositions pour le traitement des troubles de l'hémostase.

L'invention se rapporte aussi à l'utilisation de ces compositions pour utilisation comme agent cicatrisant.

Enfin, l'invention a pour objet des compositions cosmétologiques comprenant un polypeptide tel que décrit ci-dessus.

L'invention concerne des polypeptides isolés ayant la séquence du polypeptide de la SEQ ID No.1 ou du polypeptide de la position 25 à la position 152 de la SEQ ID No. 1.

Le peptide de la position 1 à la position 24 de la SEQ ID No. 1 correspond au peptide signal permettant la sécrétion de la protéine recombinante par une cellule hôte. Ce peptide signal pourra être absent ou remplacé par un autre peptide signal selon des techniques bien connues de l'homme du métier. L'homme du métier saura choisir le peptide signal homologue ou hétérologue approprié pour l'expression et la sécrétion des polypeptides de la présente invention dans différents systèmes d'expression procaryotes ou eucaryotes. De préférence, les polypeptides de la présente invention sont produits dans des cellules ou des organismes eucaryotes et en particulier dans des cellules de mammifères. Dans un mode de réalisation particulier de l'invention, les polynucléotides de la présente invention comprennent un peptide signal permettant leur sécrétion dans le milieu extracellulaire. Dans une autre mode de réalisation, l'invention se rapporte au polypeptide mature obtenu après clivage du peptide signal.

Les polypeptides de la présente invention ont une activité biologique et notamment une activité pro-agrégante sur les plaquettes sanguines humaines détectée à l'aide d'un thromboagrégomètre (société Regulest, Florange, France), selon la technique de référence (Born 1962). Le plasma riche en plaquettes (PRP) est mis en contact avec un agoniste (10 µl dans 290 µl de PRP) et l'éclaircissement du milieu (lié à la formation d'agrégats qui tombent au fond du tube) est suivi en temps réel (courbe d'agrégation). En l'absence d'agrégation plaquettaire, le signal reste plat (milieu trouble persistant). Il est possible de vérifier l'absence ou la présence d'agrégats à l'examen du tube à la fin de la mesure. L'évaluation de la réponse en tests d'agrégation plaquettaire est effectuée à 37°C, en agitation continue (1000 rpm). L'appareil est étalonné : 0% d'agrégation avec le plasma riche en plaquettes (préparation obtenue par centrifugation lente et concentration ajustée à 300 x 10⁹ / L) et 100% d'agrégation avec le plasma pauvre en plaquettes (préparation obtenue par centrifugation rapide). La qualité des préparations plaquettaires a été validée en en vérifiant la réponse à des agonistes de référence (ADP 5 µM et collagène 1 µg/mL), utilisés pour la mise au point des thérapeutiques antiplaquettaires. On considère qu'une protéine est pro-agrégante quand elle capable d'induire une agrégation supérieure à 30%, et de façon irréversible.

Les motifs peptidiques de reconnaissance de récepteurs exprimés à la surface de nombreux types cellulaires confèrent au polypeptide une activité :
- adhésion cellulaire,
- recrutement cellulaire.

L'effet pro-agrégant du polypeptide indépendant de sa structuration en triple hélice est la conséquence de l'activation d'un ou plusieurs récepteurs présents à la surface des plaquettes (α1 β2, TIIICBP et GPVI).

L'invention se rapporte également à des fragments du polypeptide de la SEQ ID No. 1 conservant au moins une des activités du polypeptide de la SEQ ID No.1. Le terme « fragment » d'un polypeptide désigne un polypeptide comprenant une partie mais pas la totalité du polypeptide dont il est dérivé. L'invention concerne ainsi un polypeptide comprenant un fragment d'au moins 100, 110, 120, 130, 140 ou 150 acides aminés du polypeptide de la SEQ ID No. 1.

Ces fragments du polypeptide de la SEQ ID No. 1 conservent au moins une des activités du polypeptide de la SEQ ID No. 1. En particulier, une activité pro-agrégante sur les plaquettes sanguines humaines. L'invention concerne donc les fragments biologiquement actifs du polypeptide de la SEQ ID No. 1. Le terme « fragment biologiquement actif» désigne un fragment d'un polypeptide conservant la fonction du polypeptide dont il est dérivé. Les fragments biologiquement actifs du polypeptide de la SEQ ID No. 1 conservent ainsi au moins une des fonctions de ce polypeptide et de préférence conservent toutes les activités biologiques du polypeptide de la SEQ ID NO. 1. Les méthodes de préparation de fragments d'un polypeptide ainsi que les techniques de mesure des activités biologiques des polypeptides de la présente invention sont bien connues de l'homme du métier.

L'invention a aussi pour objet des polypeptides ayant au moins une des activités du polypeptide de la SEQ ID No. 1 et présentant au moins 70 % d'acides aminés identiques avec le polypeptide de la SEQ ID No. 1. De préférence, ces polypeptides ont les mêmes propriétés et notamment les mêmes activités biologiques que le polypeptide de la SEQ ID No. 1. L'invention a pour objet des polypeptides présentant au moins 70%, 80%, 90%, 95%, 98% et préférentiellement au moins 99% d'acides aminés identiques avec le polypeptide de la SEQ ID No. 1. Par acides aminés identiques, on entend des acides aminés invariants ou inchangés entre deux séquences. Ces polypeptides peuvent présenter une délétion, une addition ou une substitution d'au moins un acide aminé par rapport au polypeptide de la SEQ ID No. 1.

L'invention a également pour objet des polypeptides présentant au moins 70%, 80%, 90%, 95%, 98% et préférentiellement au moins 99% d'homologie avec le polypeptide de la SEQ ID No. 1. Par homologie, on entend la mesure de la ressemblance entre séquences protéiques. Ces polypeptides peuvent présenter une délétion, une addition ou une substitution d'au moins un acide aminé par rapport au polypeptide de la SEQ ID No. 1. Le degré d'homologie entre deux séquences, quantifié par un score, est basé sur le pourcentage d'identités et/ou de substitutions conservatrices des séquences.

Les polypeptides de la présente invention présentant un certain degré d'homologie ou d'identité avec le polypeptide de la SEQ ID No .1 comportent au moins 100 ou 150 acides aminés.

Les méthodes de mesure et d'identification du degré d'identité et du degré d'homologie entre polypeptides sont connues de l'homme du métier. On peut employer par exemple Vector NTi 9.1.0, programme d'alignement AlignX (Clustal W algorithm) (Invitrogen INFORMAX, http ://www.invitrogen.com). De préférence, on utilise les paramètres par défaut.

Les polypeptides selon l'invention sont isolés ou purifiés de leur environnement naturel. Les polypeptides peuvent être préparés au moyen de différents procédés. Ces procédés sont notamment la production de polypeptides recombinants par des cellules hôtes appropriées et leur purification ultérieure, la production par synthèse chimique ou, enfin, une combinaison de ces différentes approches. Ces différents procédés de production sont bien connus de l'homme du métier. De préférence, les polypeptides de la présente invention sont produits par des cellules procaryotes ou eucaryotes recombinantes. Les polypeptides de la présente invention peuvent ainsi être produits dans des bactéries ou dans des cellules de mammifères.

L'invention a également pour objet des protéines de fusion, des protéines ou des protéines chimères comprenant les polypeptides selon l'invention. Le terme « polypeptide » désigne également des protéines ainsi que des polypeptides modifiés.

Dans un mode de réalisation de l'invention, les polypeptides selon l'invention sont glycosylés. Le polypeptide de la SEQ ID No. 1 possède notamment des sites de O-glycosylation présentant sur les acides aminés lysines présents en position 102 et 141 (en position 3 d'un triplet GXY). Dans un mode de réalisation préféré, le résidu asparagine en position 93 du polypeptide de la SEQ ID No. 1 est glycosylé.

L'invention a également pour objet des polynucléotides codant pour les polypeptides définis ci-dessus. Selon la présente invention, on entend par « polynucléotide « une chaine nucléotidique simple brin ou son complémentaire pouvant être de type ADN ou ARN, ou une chaine nucléotidique double brin pouvant être de type ADN complémentaire ou génomique. De préférence, les polynucléotides de l'invention sont de type ADN, notamment d'ADN double brin. Le terme « polynucléotide » désigne également les polynucléotides modifiés. Les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. (Molecular Cloning : A Labratory Manual, 1989) ou par synthèse chimique.

Dans un premier mode de réalisation, l'invention se rapporte au polynucléotide de la SEQ ID No. 2. Dans un deuxième mode de réalisation, l'invention se rapporte au polynucléotide de la SEQ ID No. 2. dont la séquence est comprise entre la position 73 et la position 459 de la SEQ ID No. 1. Ces polynucléotides codent pour les polypeptides définis ci-dessus.

L'invention concerne aussi des polynucléotides présentant au moins 70 %, 75%, 80%, 85%, 90%, 95%, 98% et de préférence au moins 99% d'identité avec le polynucléotide de la SEQ ID No. 2 ou avec le polynucléotide dont la séquence est comprise entre la position 73 et la position 459 de la SEQ ID No. 2. Ces polynucléotides codent de préférence pour un polypeptide conservant les activités biologiques du polypeptide de la SEQ ID No. 1.

Par nucléotides identiques, on entend des nucléotides invariants ou inchangés entre deux séquences. Ces polynucléotides peuvent présenter une délétion, une addition ou une substitution d'au moins un nucléotide par rapport au polynucléotide de référence.

L'invention concerne aussi des polynucléotides présentant au moins 70%, 75%, 80%, 85%, 90%, 95%, 98% et de préférence au moins 99% d'homologie avec le polynucléotide de la SEQ ID NO. 2 ou avec le polynucléotide dont la séquence est comprise entre la position 73 et la position 459 de la SEQ ID No. 2. Ces polynucléotides codent de préférence pour un polypeptide conservant les activités biologiques du polypeptide de la SEQ ID No. 1.

Par homologie, on entend la mesure de la ressemblance entre séquences nucléiques. Ces polynucléotides peuvent présenter une délétion, une addition ou une substitution d'au moins un nucléotide par rapport au polynucléotide de référence. Le degré d'homologie entre deux séquences, quantifié par un score, est basé sur le pourcentage d'identités et/ou de substitutions conservatrices des séquences.

Les méthodes de mesure et d'identification du degré d'identité et du degré d'homologie entre les séquences d'acides nucléiques sont bien connues de l'homme du métier. On peut employer par exemple Vector Nti Vector Nti 9.1.0, programme d'alignement AlignX (Clustal W algorithm) (Invitrogen INFORMAX, http ://www.invitrogen.com). De préférence, on utilise les paramètres par défaut.

L'invention concerne aussi des polynucléotides capables de s'hybrider de manière sélective avec le polynucléotide de la SEQ ID NO. 2 ou avec le polynucléotide dont la séquence est comprise entre la position 73 et la position 459 de la SEQ ID No. 2. De préférence, l'hybridation sélective est effectuée dans des conditions de moyenne stringence et préférentiellement dans des conditions de forte stringence. Ces polynucléotides codent de préférence pour des polypeptides ayant les activités biologiques du polypeptide de la SEQ ID No. 1. Par « séquence capable de s'hybrider de manière sélective « , on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier. En général la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant : 6X SSC, 50 mM Tris-HCI (pH 7.5), 1 mM EDTA, 0.02% PVP, 0.02% Ficoll, 0.02% BSA, 500 ug/ml sperme de saumon dénaturé DNA. Les lavages sont par exemple réalisés successivement à faible stringence dans un tampon 2X SSC, 0,1% SDS, à moyenne stringence dans un tampon 0,5X SSC, 01% SDS et à forte stringence dans un tampon 0,1X SSC, 0,1%SDS. L'hybridation peut bien entendu être effectuée selon d'autres méthodes usuelles bien connues de l'homme du métier (voir notamment Sambrook et al., Molecular Cloning : A Labratory Manual, 1989). De préférence, les polynucléotides s'hybridant de manière sélective à un polynucléotide de référence conservent la fonction de la séquence de référence.

L'invention se rapporte de manière générale aux polynucléotides codant pour les polypeptides selon l'invention. En raison de la dégénérescence du code génétique, différents polynucléotides peuvent coder pour un même polypeptide.

L'invention se rapporte aussi à des cassettes d'expression comprennent dans le sens de la transcription:
- un promoteur fonctionnel dans un organisme hôte,
- un polynucléotide selon l'invention,
- une séquence terminatrice fonctionnelle dans le même organisme hôte.

Selon un mode de réalisation de l'invention, un polynucléotide codant pour un polypeptide selon l'invention est inséré dans une cassette d'expression en utilisant des techniques de clonage bien connues de l'homme du métier. Cette cassette d'expression comprend les éléments nécessaires à la transcription et à la traduction des séquences codant pour les polypeptides selon l'invention.

Avantageusement, cette cassette d'expression comprend à la fois des éléments permettant de faire produire un polypeptide par une cellule hôte et des éléments nécessaires à la régulation de cette expression.

Tout type de séquence promotrice peut être utilisé dans les cassettes d'expression selon l'invention. Le choix du promoteur dépendra notamment de l'organisme hôte choisi pour l'expression du gène d'intérêt. Certains promoteurs permettent une expression constitutive alors que d'autres promoteurs sont au contraire inductibles. Parmi les promoteurs fonctionnels dans les bactéries, on citera notamment celui de la RNA polymérase du bacteriophage T7. Parmi les promoteurs fonctionnels dans les levures, on citera le promoteur du gène GAL1 ou les promoteurs GAL4 et ADH de *S.cervisiae* Parmi les promoteurs fonctionnels dans les cellules d'eucaryotes supérieurs et notamment dans les cellules de mammifères, on citera CMV (Cytomegalo virus), SV40, RSV (Rous Sarcoma Virus), beta-actine humaine ou de poulet, beta-globine, PGK (phosphoglycérate kinase), EF1 alpha, Thymidine Kinase, MMTV (mouse mammary tumor virus). Tous ces promoteurs sont décrits dans la littérature et bien connus de l'homme du métier.

Les cassettes d'expression, selon la présente invention, peuvent en outre inclure toute autre séquence nécessaire à l'expression des polypeptides ou des polynucléotides comme par exemple des éléments de régulation ou des séquences signal permettant la sécrétion des polypeptides produits par l'organisme hôte. On peut notamment utiliser toute séquence de régulation permettant d'augmenter le niveau d'expression de la séquence codante insérée dans la cassette d'expression. Selon l'invention, on peut notamment utiliser, en association avec la séquence de régulation promotrice, d'autres séquences de régulation, qui sont situées entre le promoteur et la séquence codante, telles que des activateurs de transcription (« enhancer »).

Une grande variété de séquences terminatrices sont utilisables dans les cassettes d'expression selon l'invention, ces séquences permettent la terminaison de la transcription et la polyadénylation de l'ARNm. Toute séquence terminatrice fonctionnelle dans l'organisme hôte sélectionné peut être utilisée.

La présente invention a également pour objet un polynucléotide comprenant une cassette d'expression selon l'invention, avantageusement les cassettes d'expression selon la présente invention sont insérées dans un vecteur.

La présente invention concerne donc également des vecteurs de réplication ou d'expression pour la transformation d'un organisme hôte comprenant au moins un polynucléotide ou une cassette d'expression selon la présente invention. Ce vecteur peut notamment correspondre à un plasmide, un cosmide, un bactériophage ou un virus dans lequel est inséré un polynucléotide ou une cassette d'expression selon l'invention. Les techniques de construction de ces vecteurs et d'insertion d'un polynucléotide de l'invention dans ces vecteurs sont bien connues de l'homme du métier. De manière générale, tout vecteur capable de se maintenir, de s'autorépliquer ou de se propager dans une cellule hôte afin d'induire notamment l'expression d'un polynucléotide ou d'un polypeptide peut être utilisé. L'homme du métier choisira les vecteurs appropriés en fonction de l'organisme hôte à transformer, et en fonction de la technique de transformation mise en oeuvre.

Les vecteurs de la présente invention sont notamment utilisés pour transformer un organisme hôte en vue de la réplication du vecteur et/ou de l'expression d'un polypeptide selon l'invention dans l'organisme hôte.

L'invention concerne aussi une méthode pour préparer un polypeptide selon l'invention comprenant les étapes suivantes :
- on transforme un organisme hôte avec un vecteur d'expression comprenant une cassette d'expression selon l'invention et/ou avec un polynucléotide selon l'invention,
- on isole les polypeptides produits par l'organisme hôte.

La présente invention a également pour objet, un procédé de transformation d'un organisme hôte par intégration dans ledit organisme hôte d'au moins un polynucléotide ou d'une cassette d'expression ou d'un vecteur selon l'invention. Le polynucléotide peut être intégré dans le génome de l'organisme hôte ou se répliquer de manière stable dans l'organisme hôte. Les méthodes de transformation des organismes hôtes sont bien connues de l'homme du métier et largement décrits dans la littérature.

La présente invention concerne également un organisme hôte transformé avec un polynucléotide, une cassette d'expression ou un vecteur selon l'invention. Par organisme hôte, on entend en particulier selon l'invention tout organisme mono ou pluricellulaire, inférieur ou supérieur, en particulier choisi parmi les bactéries, les levures et les cellules d'eucaryotes supérieurs en particulier les cellules de mammifères telles que CHO (Chinese hamster ovary cells), BHK (baby hamster kidney), HEK-293 (human embryonic kidney cell line), NSO (lignée de myélome de souris), Per.C6 (Crucell), YB2/0 (ATCC n° CRL 1662). Par organisme hôte on entend un organisme non humain.

L'invention se rapporte aussi à des compositions pour utilisation comme médicament comprenant un polypeptide selon l'invention, un polynucléotide selon l'invention, une cassette d'expression selon l'invention, un vecteur selon l'invention et/ou un organisme hôte selon l'invention. L'invention se rapporte donc également à des compositions pharmaceutiques comprenant un polypeptide selon l'invention, un polynucléotide selon l'invention, une cassette d'expression selon l'invention, un vecteur selon l'invention et/ou un organisme hôte selon l'invention.

Dans un mode de réalisation préféré, l'invention concerne des compositions pour la prévention et/ou le traitement des thromboses.

Dans un autre mode de réalisation, l'invention concerne des compositions pour la prévention ou le traitement des troubles de l'hémostase.

L'invention concerne également ces compositions pour utilisation comme agent cicatrisant.

Dans un autre mode de réalisation, l'invention des compositions pouvant également servir :
- comme réactifs de diagnostic pour détecter :
   - certaines dysfonctions plaquettaires
   - des maladies hématologiques
- comme composants actifs de pansements et colles hémostatiques dans la composition de crèmes dermatologiques et cosmétiques.

L'invention concerne aussi des méthodes de traitement thérapeutique des thromboses comprenant l'administration à un individu d'une quantité efficace d'un polypeptide selon l'invention, d'un polynucléotide selon l'invention, d'une cassette d'expression selon l'invention, d'un vecteur selon l'invention et/ou d'un organisme hôte selon l'invention.

L'invention concerne enfin l'utilisation des polypeptides, polynucléotides et organismes hôtes transformés de la présente invention pour la fabrication de médicaments.

L'invention concerne des compositions pharmaceutiques comprenant un polypeptide, un polynucléotide ou un organisme hôte transformé tels que définis dans la présente invention et un véhicule pharmaceutique approprié.

Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

L'invention concerne aussi des compositions cosmétologiques comprenant un polypeptide tel que décrit ci-dessus.

Ces compositions peuvent en outre comprendre des actifs classiquement utilisés en dermatologie tels que les émollients, les actifs hydratants, les agents restructurants de la barrière cutanée, les agents anti-irritants, les agents apaisants. Les compositions cosmétologiques selon l'invention peuvent être formulées sous la forme de différentes préparations adaptées à une administration topique. Selon un mode de réalisation préféré, les différentes préparations sont adaptées à l'administration topique et incluent les crèmes, les émulsions, les laits, les pommades, les lotions, les huiles, les solutions aqueuses ou hydro -alcooliques ou glycoliques, les poudres, les sprays, les gelées, les gels, hydrogels ou tout autre produit pour application externe. Ces compositions cosmétiques peuvent également des antioxydants, des conservateurs, etc. L'invention concerne en outre une méthode de traitement cosmétique, de soin hygiénique, d'embellissement et/ou une méthode pour parfumer des muqueuses et/ou des peaux normales, sèches, grasses, mixtes, déshydratées, âgées, sensibles, irritées, inconfortables, intolérantes, présentant un déséquilibre lié au vieillissement intrinsèque, extrinsèque ou hormonal ou lié aux agressions exogènes (polluants, UV, stress...), à tendance allergique, présentant des troubles de la pigmentation, caractérisée en ce qu'elle consiste à appliquer une composition selon l'invention.

### FIGURES

- Figure 1 :: Expression des ARN messagers codant pour la protéine dans des cellules CHO-S adhérentes transfectées de façon transitoire.
- Figure 2 :: La figure 2 représente l'agrégation plaquettaire induite par du milieu conditionné de cellules CHO-S adhérentes, transfectées de façon transitoire, en présence ou non d'épinéphrine.
- Figure 3 :: La figure 3 représente l'expression des ARN messagers codant pour la protéine dans un clone cellulaire stable.
- Figure 4 :: La figure 4 montre la détection par Western blotting de la présence de la protéine-6X-histidine dans les différentes fractions bactériennes obtenues après purification.
- Figure 5 :: La figure 5 montre l'agrégation plaquettaire induite par la protéine produite en bactérie.
- Figure 6 :: La figure 6 montre la validation par Western blotting de la spécificité du sérum de lapin obtenu après 89 jours d'immunisation.

### EXEMPLES

### A. Production transitoire par des cellules de mammifères

### 1. Construction des vecteurs

### Plasmide 1 (NVH001-A)

Le plasmide *NVH001-A* contient les éléments suivants :
- un intron provenant du plasmide pCInéo (Promega : pCIneo Mammalian Expression Vector)
- notre protéine d'intérêt NVH001 provenant du vecteur pUC57-NVH001 (commande à genecust) et possédant le peptide signal du collagène de type III (NP_000081)
- une queue polyA provenant de l'ARNm de l'hGH (N_000515)
- un promoteur CMV provenant de pCDF1-MCS1-EF1-copGFP (System Biosciences : pCDF cDNA cloning and Expression Lentivectors).

### Plasmide 2 (NVH001-B1), 3 (NVH001-B2), 4 (NVH001-Cl) et 5 (NVH001-C2₎

Les plasmides *NVH001-B1* et *NVH001-C1* contiennent les éléments suivants :
- une origine SV40 provenant du plasmide dérivé pSV2-Neo (ori SV40 :1-989 dans le vecteur, ATCC 37149)
- un promoteur/enhancer pouvant être l'un de ceux décrits dans Molecular Cloning: a Laboratory Manual: 3rd edition, Sambrook and Russell, 2001, Cold Spring Harbor Laboratory Press
- une cassette hygromycine provenant de pMono-hygro-mcs (Invivogen)
- notre protéine d'intérêt NVH001 provenant du vecteur pUC57-NVH001 (commande à Genecust) et possédant le peptide signal du collagène de type III qui_000081)
- un propeptide C provenant du plasmide pUC57-proC (commande à Genecust)
- une queue polyA provenant de l'ARNm de l'hGH (NM_000515).

Les plasmides *NVH001-B2* et *NVH001-C2* contiennent les mêmes éléments que les plasmides *NVH001-B1* et *Cl* sauf le propeptide C.

Tous les plasmides finaux sont séquencés pour vérifier qu'aucune mutation ne s'est introduite dans l'ADN de la protéine d'intérêt et dans les éléments apportés.

### 2. Transfection de cellules de mammifères

### Plasmide 1 (NVH001-A)

1. Le vecteur *NVH001-A* est transfecté, de façon transitoire, dans des cellules CHO-S rendues adhérentes et cultivées dans du milieu RPMI contenant 5 % de sérum de veau foetal à l'aide du kit lipofectamine 2000 d'Invitrogen.
2. Les surnageants de culture sont collectés aux jours 3 et 4, les protéines produites sont purifiées puis leur activité de liaison aux plaquettes est testée à l'aide d'un agrégomètre.

### Plasmides 2 (NVH001-B1), 3 (NVH001-B2), 4 (NVH001-C1) et 5 (NVH001-C2)

1. Les vecteurs *NVH001-B1, NVH001-B2, NVH001-C1* et *NVH001-C2* sont transfectés de façon transitoire dans des cellules CHO-S rendues adhérentes et cultivées dans du milieu RPMI contenant 5 % de sérum de veau foetal à l'aide du kit lipofectamine 2000 d'invitrogen.

Les ARN des cellules transfectées sont extraits aux jours 1, 2, 3, 4 et 5 par ajout de 800 µL de TRIZOL (In vitrogen). Les ADNc correspondants sont obtenus par transcription inverse à partir de 1 µg d'ARN totaux. Une amplification par PCR a été réalisée sur les ADNc. Les amplicons sont ensuite détectés après migration sur un gel d'agarose 2% pendant 30 minutes à 125V. Un témoin positif (plasmide transfecté) et un témoin négatif ont également été réalisés. Le marqueur de poids moléculaire (à gauche) permet de vérifier la taille des amplicons.

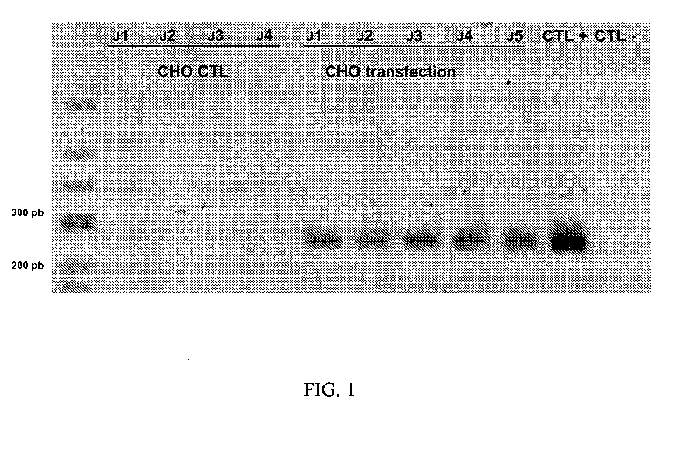

On observe l'expression dans les cellules transfectées de l'ARNm codant pour notre protéine dès le jour 1. Cette expression augmente légèrement au cours du temps et est maintenue après 5 jours. Le suivi de l'expression des ARNm est représenté à la figure 1.

2. Les surnageants de culture sont collectés aux jours 3 et 4, les protéines produites sont purifiées puis leur activité de liaison aux plaquettes est testée à l'aide d'un agrégomètre.

### 3. Activité biologique de notre protéine : agrégation plaquettaire

L'activité de liaison de notre protéine aux plaquettes est évaluée par agrégométrie. Des prélèvements sanguins en tube citrate, collectés chez des donneurs sains volontaires après signature d'un consentement, sont centrifugés à 150g pendant 15 minutes afin d'obtenir un plasma riche en plaquettes (PRP). La concentration est ajustée à 300 giga/L. Les tests d'agrégation sont réalisés à l'aide d'un thromboagrégomètre Régulest^{®}, sous agitation (1000 rpm) à 37°C. Cette technique est basée sur la lecture photométrique selon la méthode de Born qui consiste à mesurer l'évolution de la transmission lumineuse d'un milieu trouble, le PRP, en réponse à un activateur de la fonction plaquettaire. En cas de formation d'agrégats plaquettaires, le milieu s'éclaircit et on observe une augmentation de la transmission lumineuse. On considère qu'une molécule a un effet pro-agrégant lorsque l'on observe une augmentation de plus de 30% de cette transmission associée à une cassure de la pente correspondant à cet éclaircissement.

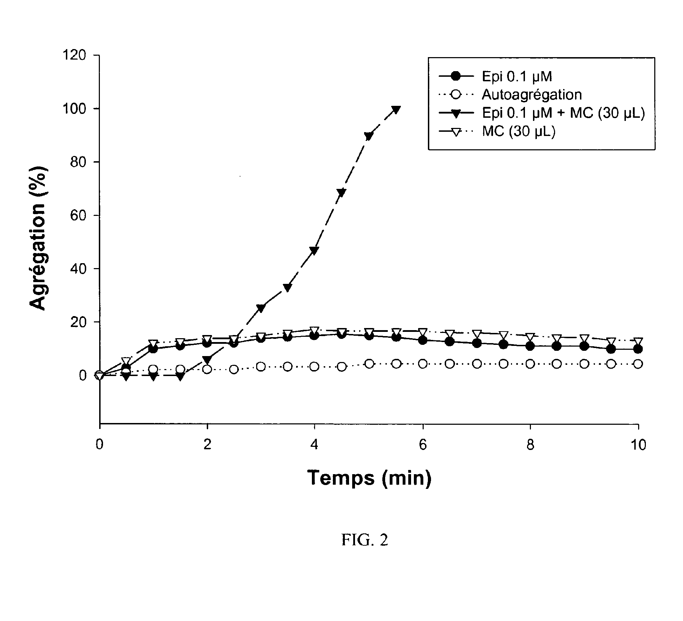

La figure 2 représente l'agrégation plaquettaire induite par du milieu conditionné de cellules CHO-S adhérentes, transfectées de façon transitoire, en présence ou non d'épinéphrine.

Le mélange réactionnel est composé de 290 µL de PRP auquel est ajouté 30 µL de milieu conditionné en présence ou non de 0,1 µM d'épinéphrine. La mesure de la réponse plaquettaire est suivie pendant 10 minutes. L'autoagrégation (-O-) qui défini la ligne de base. L'ajout d'épinéphrine à 0,1 µM induit un léger décalage de la ligne de base, stable pendant les 10 minutes (-●-). L'ajout de 30 µL de milieu conditionné induit une réponse limitée (18%) après 2 minutes (-∇-). ce décalage est stable pendant les 10 minutes d'observation. L'ajout d'épinéphrine pendant 2 minutes, suivi de l'ajout de 30 µL de milieu conditionné induit une agrégation totale des plaquettes en 5 minutes (-▼-). Cette agrégation n'est pas réversible suggérant que les agrégats formés sont stables.
L'ajout de 30 µL de milieu conditionné de cellules CHO-S adhérentes non transfectées n'induit pas d'agrégation (résultat non présenté).

### B. Production stable par des cellules de mammifères

### 1. Constructions des vecteurs

### Plasmide 1 (NVH001-A)

1. L'ensemble intron-NVH001-hGHpA est retiré du vecteur construit pour l'expression transitoire et est cloné dans un vecteur de production contenant une cassette hygromycine provenant de pMono-hygro-mcs (Invivogen).
2. Le vecteur final est séquencé pour vérifier qu'aucune mutation ne s'est introduite dans l'ADN de la protéine d'intérêt et dans les éléments apportés.
3. Le vecteur linéarisé est transfecté de façon stable dans des cellules CHO rendues adhérentes à l'aide du kit lipofectamine 2000 d'invitrogen. Les cellules transfectées sont sélectionnées par paliers progressifs d'hygromycine B.

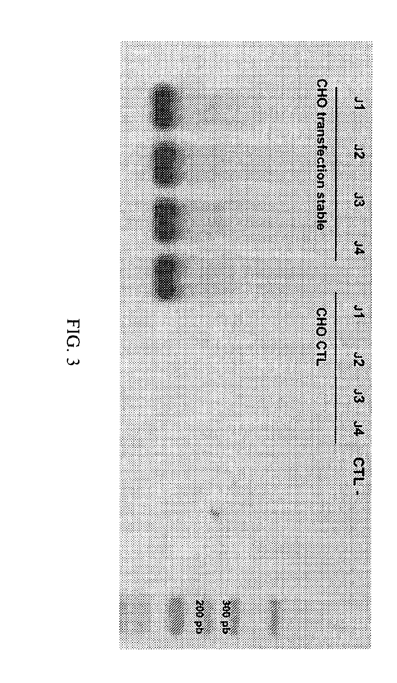

La figure 3 représente l'expression des ARN messagers codant pour la protéine dans un clone cellulaire stable.

Les ARN des cellules sont extraits aux jours 1, 2, 3 et 4 par ajout de 800 µL de TRIZOL (In vitrogen). Les ADNc correspondants sont obtenus par transcription inverse à partir de 1 µg d'ARN totaux. Une amplification par PCR a été réalisée sur les ADNc à l'aide des primers 5'-AGCTGGCGCGCCGCCACCATG-3' (S) et 5'-GCTTCCGGGAGGCCCTGGCTTCCCATC-3' (AS). Les amplicons sont ensuite détectés après migration sur un gel d'agarose 2% pendant 30 minutes à 125V. Un témoin positif, correspondant à l'ADN plasmidique est utilisé. Le marqueur de poids moléculaire (à droite) permet de vérifier la taille des amplicons. On observe une expression stable dans le clone cellulaire.

### C. Production en bactéries

### 1. Construction

L'ADN de la molécule d'intérêt (NVH001) est amplifiée par PCR sur le vecteur pUC57-NVH001 à l'aide des amorces tagNVH001-sens : GCTGCCATGGGCAGCAGCCATCATCATCATCATCACGGTCGCCCGGGAGCTCC TGGAGAGAGAGGATTG et tagNVH001-antisens : GCACGGATCCTATTAGCCA GGGCAAGGTCCAGGGGCTC qui permettent de rajouter un tag-6X histidine du côté N-terminal de la protéine NVH001. Les produits PCR sont clonés dans le vecteur pET3d (pET3d-NVH001) permettant la production de la molécule en bactéries (New England Biolab).

La présence et la séquence correcte de l'ADN sont vérifiées par séquençage.

### 2. Production

1. Le vecteur pET3d-NVH001 est transfecté dans des bactéries BL21 permettant la production en masse de protéine.
2. Les bactéries sont inoculées dans 1 L de milieu nutritif et incubées à 37°C jusqu'à obtenir une DO600nm où les bactéries sont en phase exponentielle.
3. La production de la protéine est induite par de l'IPTG pendant 5 heures 30°C.
4. Les bactéries sont culottées, lysées et notre protéine est purifiée dans la fraction soluble (surnageant) et insoluble (corps d'inclusion) des bactéries sur des colonnes antitag-6Xhistidine de Macherey-nagel.

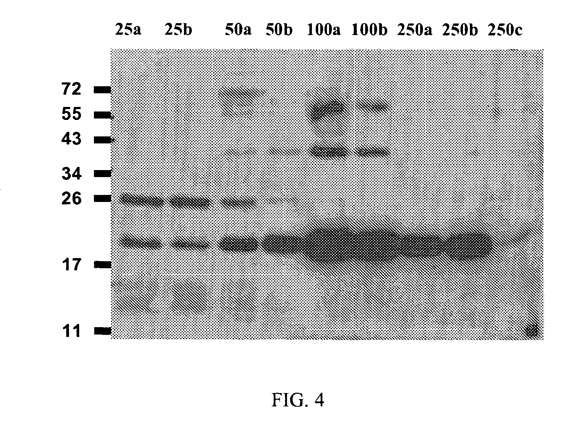

La figure 4 montre la détection par Western blotting de la présence de la protéine-6X-histidine dans les différentes fractions bactériennes obtenues après purification.

40 µL de chaque fraction obtenue après élution de la colonne anti-tag-6Xhistine est déposé sur un gel à 16 % de polyacrylamide. L'électrophorèse est effectuée dans du tampon de migration (192 mM de glycine, 25 mM de Tris-HCl pH 6,8 et 0,1% SDS) à 18 mA, en présence de marqueurs de poids moléculaires (Amersham) à l'aide du système de migration Mini-Protean 3 (Biorad). Les protéines séparées dans le gel sont transférées sur une membrane de PVDF (Biorad) pendant 60 min à 70 V. Les fixations non-spécifiques sont bloquées par incubation de la membrane 120 min à 37°C et sous agitation, dans une solution saline de Tris + 0,1 % de Tween-20 (TBS-T) contenant 5 % de lait écrémé. La membrane est ensuite incubée une nuit à 4°C avec un anticorps monoclonal anti-tag-6Xhistine (Cell Signaling) dilué au 1/1000° dans du TBS-T. Après 3 lavages de 5 min dans du TBS-T, la membrane est incubée 60 min à température ambiante dans un anticorps anti-souris couplé à la peroxydase (Jackson Laboratories) dilué au 1/10000^{ème}. La membrane est à nouveau lavée trois fois 5 min dans du TBS. La révélation est obtenue par la technique de chimioluminescence à l'aide du kit ECL (Amersham).

On observe la présence de la protéine dans les différentes fractions. La bande principale obtenue à un poids moléculaire autour des 19 kDa correspondant à la forme monomérique de la protéine. On peut également observer la présence dans certaines fractions de bandes correspondant à des protéines de poids moléculaires 26, 43 et 60 kDa.

### 3. Démonstration de son activité biologiques : agrégation plaquettaire

L'activité de liaison de notre protéine aux plaquettes est évaluée par agrégométrie. Des prélèvements sanguins en tube citrate, collectés chez des donneurs sains volontaires après signature d'un consentement, sont centrifugés à 150g pendant 15 minutes afin d'obtenir un plasma riche en plaquettes (PRP). La concentration est ajustée à 300 giga/L. Les tests d'agrégation sont réalisés à l'aide d'un thromboagrégomètre Régulest^{®}, sous agitation (1000 rpm) à 37°C. Cette technique est basée sur la lecture photométrique selon la méthode de Born qui consiste à mesurer l'évolution de la transmission lumineuse à travers le PRP, qui est au départ un milieu trouble, en réponse à un activateur plaquettaire. En cas de formation d'agrégats plaquettaires, le milieu s'éclaircit et on observe une augmentation de la transmission lumineuse. On considère qu'une molécule a un effet pro-agrégant lorsque l'on observe une augmentation de plus de 30% de cette transmission associée à une cassure de la pente correspondant à cet éclaircissement.

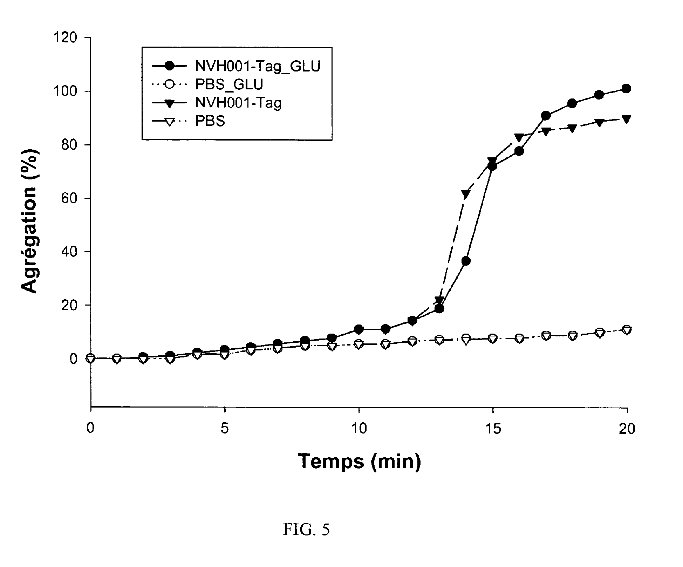

La figure 5 montre l'agrégation plaquettaire induite par la protéine produite en bactérie.

Les différentes fractions éluées à partir de la colonne anti-tag-6Xhistine contenant la protéine détectée par western-blotting sont regroupées et un lyophilisat est obtenu par centrifugation. Une partie de ce lyophilisat est incubée en présence de 0,25 % de glutaraldéhyde pendant 3 heures à 4°C puis dialysée une nuit contre du PBS 1X à 4°C avant son utilisation en agrégométrie.

Le mélange réactionnel est composé de 290 µL de PRP auquel est ajouté 30 µL de PBS contenant la protéine. La mesure de la réponse plaquettaire est suivie pendant 25 minutes. Les tracés -○- et -V- montrent la réponse obtenue après ajout d'une solution de PBS et de PBS-glutaraldéhyde dialysée, respectivement : aucune agrégation n'est observée. L'ajout de PBS contenant la protéine non agrégée par le glutaraldéhyde (-▼-) ou agrégée par le glutaraldéhyde et dialysée (-●-) montre une agrégation importante 10 minutes après leur ajout. Cette agrégation atteint respectivement 90% (-▼-) et 100% (-●-) après 20 minutes.

### D. Production d'anticorps anti-protéine d'intérêt chez le lapin

1. La protéine est produite en grande quantité dans des bactéries BL21 comme précédemment décrit.
2. Deux lapins sont immunisés 4 fois à 3 semaines d'intervalle avec la protéine purifiée additionnée (v/v) d'adjuvant de Freund, soit à J0, J21, J42 et J63.
3. Les lapins sont sacrifiés en fin de protocole à J89 et les sérums sont récupérés par centrifugation après exsanguination des animaux.
4. Les IgG sont purifiés par chromatographie d'affinité sur une colonne spécifique afin d'éliminer les protéines plasmatiques.

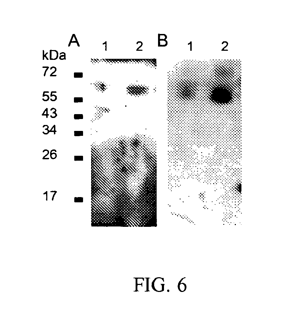

La figure 6 montre la validation par Western blotting de la spécificité du sérum de lapin obtenu après 89 jours d'immunisation.

10 µL de deux préparations de protéines produites en bactéries, dont l'activité pro-agrégante a été validée par agrégométrie, est déposé sur un gel à 16 % de polyacrylamide. L'électrophorèse est effectuée dans du tampon de migration (192 mM de glycine, 25 mM de Tris-HCl pH 6,8 et 0,1% SDS) à 18 mA, en présence de marqueurs de poids moléculaires (Amersham) à l'aide du système de migration Mini-Protean 3 (Biorad). Les protéines séparées dans le gel sont transférées sur une membrane de PVDF (Biorad) pendant 60 min à 70 V. Les fixations non-spécifiques sont bloquées par incubation de la membrane 120 min à 37°C et sous agitation, dans une solution saline de Tris + 0,1 % de Tween-20 (TBS-T) contenant 5 % de lait écrémé. La membrane est ensuite incubée une nuit à 4°C soit avec du sérum de lapin, prélevé au jour 89 du protocole d'immunisation et dilué au 1/100', soit avec un anticorps monoclonal anti-tag-6Xhistine (Cell Signaling) dilué au 1/1 000^{e} dans du TBS-T. Après 3 lavages de 5 min dans du TBS-T, la membrane est incubée 60 min à température ambiante dans un anticorps anti-souris ou anti-lapin couplé à la peroxydase (Jackson Laboratories) dilué au 1/10 000^{ème}. La membrane est à nouveau lavée trois fois 5 min dans du TBS. La révélation est obtenue par la technique de chimioluminescence à l'aide du kit ECL (Amersham).

La révélation montre la présence d'une bande double, présente dans les deux préparations protéiques (A, puits 1 et 2), d'un poids moléculaire de 60 kDa. Cette bande est également détectée par l'anticorps anti-tag-6Xhistine montrant qu'il s'agit bien de notre protéine (B, puits 1 et 2). A noter que nous n'observons plus les autres bandes détectées par l'anticorps anti-tag-6Xhistine sur les fractions purifiées de bactéries. Ce résultat suggère que l'immunisation n'a été efficace que pour la forme multimérique de notre protéine ou que notre protéine s'assemble en un multimère une fois purifiée.

### REFERENCES

Asselin et coll., Biochem J. 1999 Apr 15; 339 (Pt 2) : 413-8
Born et coll, Nature 1962 June, 194: 927-9
Boudko et Engel, J Mol Biol. 2004 Jan 30; 335(5) : 1289-97
Bulleid et al., EMBO J. 1997 Nov 17; 16(22) : 6694-701
Bulleid et coll., Biochem J. 1996 Jul 1; 317 (Pt 1) : 195-202
Farndale et coll., Biochem Soc Trans. 2008 Apr; 36 (Pt 2) : 241-50
Hulmes DJ, J Struct Biol. 2002 Jan-Feb; 137(1-2) : 2-10
Jarvis et coll., Blood. 2008 May 15; 111 (10) : 4986-96
Kim et coll., J Biol Chem. 2005 Sep 16; 280(37) : 32512-20
Lisman T et coll., Blood. 2006 Dec 1; 108(12) : 3753-6
McAlinden et coll., J Biol Chem. 2003 Oct 24; 278(43) : 42200-7
Monnet E et coll., J Biol Chem. 2000 Apr 14; 275(15) : 10912-7
Morton et coll., Biochem J. 1995 Mar 1; 306 (Pt 2) : 337-44
Olsen et coll., Adv Drug Deliv Rev. 2003 Nov 28; 55(12) : 1547-67
Pires et coll., Eur J Med Chem. 2007 May; 42(5) : 694-701
Raynal et coll., J Biol Chem. 2006 Feb 17; 281(7) : 3821-31
Ruggiero et Koch, Methods. 2008 May; 45(1) : 75-85
Smethurst et coll., J Biol Chem. 2007 Jan 12; 282(2) : 1296-304
Verkleij et coll., Blood. 1998 May 15; 91(10) : 3808-16

## Revendications

1. Polypeptide isolé **caractérisé en ce qu'**il a la séquence du polypeptide de la SEQ ID No.1 ou du polypeptitle de la position 25 à la position 152 de la SEQ ID No. 1.

2. Polypeptide isolé **caractérisé en ce que** :
- il présente au moins 70% d'identité sur toute sa longueur avec le polypeptide de la SEQ ID No.1 ou avec le polypeptide de la position 25 à la position 152 de la SEQ ID No. 1,
- il est capable d'induire une agrégation des plaquettes sanguines humaines supérieure à 30% dans un thromboagrégomètre à 37°C avec une agitation de 1000 rpm.

3. Polypeptide isolé selon la revendication 2 **caractérisé en ce qu'**il comprend au moins les motifs peptidiques suivants :
- GX₁X₂GER dans lequel X₁ et X₂ représentent indépendamment un acide aminé choisi parmi A, R, N, D, Q, E, G, H, 1, K, M, F, P, S, T, W, Y, V et O ;
- (GPX₃)ₙ avec n compris entre 4 et 10 et X₃ représente P ou O ;
- GPRGQX₄GVMGFX₅ où X₄ et X₅ représentent indépendamment P ou O.

4. Polypeptide isolé selon la revendication 2 **caractérisé en ce qu'**il comprend au moins les motifs peptidiques suivants :
- GAPGER,
- KPGEPGPK,
- (GPP)ₙ avec n compris entre 4 et 10,
- RGD.

5. Polynucléotide isolé **caractérisé en ce qu'**il code pour un polypeptide selon l'une des revendications 1-4,

6. Polynucléotide isolé selon la revendication 5 **caractérisé en ce qu'**il a la séquence du polynucléotides de la SEQ ID No. 2.

7. Cassette d'expression comprennent dans le sens de la transcription:
- un promoteur fonctionnel dans un organisme hôte,
- un polynucléotide selon l'une des revendications 5-6;
- une séquence terminatrice fonctionnelle dans le même organisme hôte.

8. Vecteur **caractérisé en ce qu'**il comprend un polynucléotide selon l'une des revendications 5-6 et/ou une cassette d'expression selon la revendication 7.

9. Organisme hôte, à l'exclusion de l'homme, transformé avec un polynucléotide selon l'une des revendications 5-6, une cassette d'expression selon la revendication 7 et/ou un vecteur selon la revendication 8.

10. Composition pour utilisation comme médicament **caractérisée en ce qu'**elle comprend un polypeptide selon l'une des revendications 1-4, un polynucléotide selon l'une des revendications 5-6, une cassette d'expression selon la revendication 7, un vecteur selon la revendication 8 et/ou un organisme hôte selon la revendication 9.

11. Composition pour utilisation comme médicament selon la revendication 10 pour le traitement des maladies thrombotiques.

12. Composition pour utilisation comme médicament selon la revendication 10 pour le traitement des troubles de l'hémostase.

13. Composition selon la revendication 10 pour utilisation comme agent cicatrisant.

14. Composition cosmétologique **caractérisée en ce qu'**elle comprend un polypeptide selon l'une des revendications 1-4.

## Claims

1. Isolated polypeptide **characterised in that** it has the sequence of the polypeptide of SEQ ID No. 1 or the polypeptide of position 25 to position 152 of SEQ ID No. 1.

2. Isolated polypeptide **characterised in that**:
- it presents at least 70% identity over the entire length thereof with the polypeptide of SEQ ID No. 1 or with the polypeptide of position 25 to position 152 of SEQ ID No. 1,
- it is capable of inducing an aggregation of human blood platelets greater than 30% in a platelet aggregometer at 37°C under shaking of 1000 rpm.

3. Isolated polypeptide according to claim 2 **characterised in that** it comprises at least the following peptide motifs:
- GX₁X₂GER wherein X₁ and X₂ represent independently an amino acid selected from A, R, N, D, Q, E, G, H, I, K, M, F, P, S, T, W, Y, V and O;
- (GPX₃)ₙ with n comprised between 4 and 10 and X₃ representing P or O;
- GPRGQX₄GVMGFX₅ where X₄ and X₅ represent independently P or O.

4. Isolated polypeptide according to claim 2 **characterised in that** it comprises at least the following peptide motifs:
- GAPGER,
- KPGEPGPK,
- (GPP)n with n comprised between 4 and 10,
- RGD.

5. Isolated polynucleotide **characterised in that** it codes for a polypeptide according to any of the claims 1-4.

6. Isolated polynucleotide according to claim 5 **characterised in that** it has the sequence of the polynucleotide of SEQ ID No. 2.

7. Expression cassette comprising in the direction of transcription:
- a promoter functional in a host organism,
- a polynucleotide according to any of claims 5-6;
- a termination sequence functional in the same host organism.

8. Vector **characterised in that** it comprises a polynucleotide according to any of claims 5-6 and/or an expression cassette according to claim 7.

9. Host organism, excluding humans, transformed with a polynucleotide according to any of claims 5-6, an expression cassette according to claim 7 and/or a vector according to claim 8.

10. Composition for use as a drug **characterised in that** it comprises a polypeptide according to any of claims 1-4, a polynucleotide according to any of claims 5-6, an expression cassette according to claim 7, a vector according to claim 8 and/or a host organism according to claim 9.

11. Composition for use as a drug according to claim 10 for the treatment of thrombotic diseases.

12. Composition for use as a drug according, to claim 10 for the treatment of haemostasis disorders.

13. Composition according to claim 10 for use as a cicatrisation agent.

14. Cosmetic composition **characterised in that** it comprises a polypeptide according to any of claims 1-4.

## Patentansprüche

1. Isoliertes Polypeptid, **dadurch gekennzeichnet, dass** es die Sequenz des Polypeptids von SEQ ID No. 1 oder des Polypeptids von der Position 25 bis zur Position 152 von SEQ ID No. 1 aufweist.

2. Isoliertes Polypeptid, **dadurch gekennzeichnet, dass**
- es mindestens 70% Identität auf seiner gesamten Länge mit dem Polypeptid von SEQ ID No. 1 oder mit dem Polypeptid von der Position 25 bis zur Position 152 von SEQ ID No. 1 aufweist,
- es in der Lage ist, eine Aggregation von humanen Blutplättchen von mehr als 30% in einem Thromboaggregometer bei 37 °C mit einer Agitation von 1000 Upm zu induzieren.

3. Isoliertes Polypeptid nach Anspruch 2, **dadurch gekennzeichnet, dass** es mindestens die folgenden Peptidmotive umfasst:
- GX₁X₂GER, wobei X₁ und X₂ unabhängig voneinander eine Aminosäure repräsentieren, die ausgewählt ist aus A, R, N, D, Q, E, G, H, I, K, M, F, P, S, T, W, Y, V und O;
- (GPX₃)ₙ mit n zwischen 4 und 10, wobei X₃ P oder O repräsentiert;
- GPRGQX₄GVMGFX₅, wobei X₄ und X₅ unabhängig voneinander P oder O repräsentieren.

4. Isoliertes Polypeptid nach Anspruch 2, **dadurch gekennzeichnet, dass** es mindestens die folgenden Peptidmotive umfasst:
- GAPGER,
- KPGEPGPK,
- (GPP)ₙ mit n zwischen 4 und 10,
- RGD.

5. Isoliertes Polynukleotid, **dadurch gekennzeichnet, dass** es für ein Polypeptid nach einem der Ansprüche 1 bis 4 kodiert.

6. Isoliertes Polynukleotid nach Anspruch 5, **dadurch gekennzeichnet, dass** es die Sequenz des Polynukleotids von SEQ ID No. 2 aufweist.

7. Expressionskassette, umfassend in Transkriptionsrichtung:
- einen Promotor, der in einem Wirtsorganismus funktionell ist,
- ein Polynukleotid nach einem der Ansprüche 5 bis 6;
- eine Terminationssequenz, die in demselben Wirtsorganismus funktionell ist.

8. Vektor, **dadurch gekennzeichnet, dass** er ein Polynukleotid nach einem der Ansprüche 5 bis 6 und/oder eine Expressionskassette nach Anspruch 7 umfasst.

9. Wirtsorganismus, mit Ausnahme des Menschen, der mit einem Polynukleotid nach einem der Ansprüche 5 bis 6, einer Expressionskassette nach Anspruch 7 und/oder einem Vektor nach Anspruch 8 transformiert ist.

10. Zusammensetzung für die Verwendung als Medikament, **dadurch gekennzeichnet, dass** sie ein Polypeptid nach einem der Ansprüche 1 bis 4, ein Polynukleotid nach einem der Ansprüche 5 bis 6, eine Expressionskassette nach Anspruch 7, einen Vektor nach Anspruch 8 und/oder einen Wirtsorganismus nach Anspruch 9 umfasst.

11. Zusammensetzung für die Verwendung als Medikament nach Anspruch 10 für die Behandlung von thrombotischen Erkrankungen.

12. Zusammensetzung für die Verwendung als Medikament nach Anspruch 10 für die Behandlung von Hämostasestörungen.

13. Zusammensetzung nach Anspruch 10 für die Verwendung als narbenbildender Wirkstoff.

14. Kosmetologische Zusammensetzung, **dadurch gekennzeichnet, dass** sie ein Polypeptid nach einem der Ansprüche 1 bis 4 umfasst.
